# EUROPÄISCHE PATENTSCHRIFT

(11) **EP 2 136 760 B1**
(45) Veröffentlichungstag und Bekanntmachung des Hinweises auf die Patenterteilung: **22.09.2010**
(21) Anmeldenummer: 08714770.8
(22) Anmeldetag: 19.03.2008
(51) Int. Cl.: A61G 13/12

(54) **UNTERLAGE ZUM LAGERN EINES PATIENTEN**
SUPPORT FOR SUPPORTING A PATIENT
SUPPORT POUR LE POSITIONNEMENT D'UN PATIENT

(30) Priorität: 20.03.2007 CH 440072007
(43) Veröffentlichungstag der Anmeldung: 30.12.2009
(73) Patentinhaber: Samarit Medizintechnik Ag, 8126 Zumikon (CH)
(72) Erfinder: ALBRECHT, Willi, CH-8854 Galgenen (CH)
(74) Vertreter: Felder, Peter
(86) Internationale Anmeldenummer: PCT/CH2008/000120
(87) Internationale Veröffentlichungsnummer: WO 2008/113200

(56) Entgegenhaltungen:
- WO-A-02/28338
- WO-A-03/077821
- US-A- 4 688 780
- US-A- 5 147 287
- US-A- 5 337 760

## Beschreibung

### Technisches Gebiet

Die Erfindung betrifft eine Unterlage zum Lagern eines Patienten mit einem Kopfteil und einem Körperteil.

### Stand der Technik

Zur Operation oder Untersuchung von Patienten im oberen Körperbereich, z.B. im Kiefer- oder Schulterbereich sind zahlreiche Auflagemittel bekannt. Solche Auflagemittel können die entweder auf einer entsprechenden Patientenliege aufgearbeitet sind oder auf diesen z.B. für die spezielle Operation befestigt werden. Eine typische Patientenliege mit einem Kopfteil ist aus der DE 200 09 909 U1 bekannt, bei der auch vorgeschlagen wird, den Kopfteil mit einem gepolsterten Kissenteil zu versehen.

Den bekannten Lagerungshilfen für Patienten ist es aber gemein, dass zwar der Kopfbereich und der Rückenbereich des Patienten unterstützt werden, es fehlt aber offenbar an einer wirksamen Unterstützung des Nackenbereichs. Dies ist beim Stand der Technik erheblicher Mangel, insbesondere, wenn sich der Patient bei der Behandlung (Operation) in einen narkotisierten Zustand befindet und aus diesem Grund die Nackenmuskulatur nicht angespannt ist. Weiterhin hat sich die fehlende Unterstützung des Nackenbereichs des Patienten, insbesondere bei Operationen des Schulterbereichs in einer sogenannten "beach chair" Position, als problematisch herausgestellt, wenn - um narkotisierten Zustand des Patienten - dieser bewegt werden muss. Einerseits kann es - mit den Lagerungsmitteln gemäss dem Stand der Technik - zu Verrenkungen kommen, die vom Patienten postoperativ als sehr störend empfunden werden. In schlimmeren Fällen kann es - ohne eine wirksame Nackenunterstützung sogar zu Verletzungen z.B. im Wirbelbereich kommen, wenn der Patient ruckartigen Bewegungen ausgesetzt wird.

Bei einer Unterstützung des Nackenbereich tritt allerdings das Problem auf, dass die gesamte Einheit, die an einem Ende - normalerweise etwas überhöht - mit einem Kopfteil beginnt und am anderen Ende mit dem Rückenteil endet der Körperform des Patienten entsprechen muss, was die Ausbildung eines Nackenteiles schwierig erscheinen lässt.

### Darstellung der Erfindung

Aufgabe der Erfindung ist es, eine Unterlage zum Lagern eines Patienten mit einem Kopfteil und einem Körperteil vorzuschlagen, mit der die oben genannten Mängel am Stand der Technik behoben werden können und die zudem möglichst universell einsetzbar ist. In jedem Fall soll eine "beach chair" Position für chirurgische Eingriffe im Schulterbereich des Patienten ermöglicht werden. Weiterhin soll es ermöglicht werden, dass z.B. beim Intubieren des Patienten die dazu notwendige Veränderung der Körperposition eingestellt wird.

Die Aufgabe der Erfindung wird mit einer Unterlage nach Anspruch 1 gelöst. Dabei haben die Massnahmen der Erfindung zunächst einmal zur Folge, dass die fehlende Unterstützung im Nackenbereich gewährleistet werden kann, ohne dass dies zu einer für den Nackenbereich negativen harten Auflage führt. Weiterhin haben die Massnahmen der Erfindung zur Folge, dass der gesamte Oberkörper des Patienten bei vom Operateur veranlassten Bewegungen sicher aufliegt, was bei den Geräten gemäss dem Stand der Technik nicht umfassend möglich ist. Überraschenderweise hat sich herausgestellt, dass die beiden Auflageteile für den Kopf und den Hals so miteinander zusammenwirken können, dass bestimmte vorteilhafte Positionsänderungen des Kopfes, z.B. zum Intubieren des Patienten, herbeigeführt werden können. Die Unterlage ermöglicht die Verwendung in den verschiedensten Positionen von der liegenden über die halbsitzende bis zu sitzenden Beach Chair Position des Patienten.

Vorteilhafterweise werden die Dimensionen an den typischen menschlichen Körperbau von Patienten angepasst, die auf einer Unterlage gemäss der vorliegenden Erfindung operiert oder auch anders behandelt werden sollen (Anspruch 2 und 3). Dies kann zu unterschiedlichen Ausführungen führen, wenn z.B. eine Unterlage für Kinder, für erwachsene Patienten mit Durchschnittsmassen oder für Patienten mit einer Übergrösse hergestellt werden soll. Besonders vorteilhaft ist die Unterlage gemäss der vorliegenden Erfindung für Schulteroperationen, wobei der Tisch in die sogenannte "beach chair" Position aufgesetzt wird.

Für eine einfache Herstellung und Handhabung kann es vorteilhaft sein, wenn bei der Unterlage mindestens der Rückenteil, der Thoraxteil und der Halsteil, vorzugsweise aber auch der Kopfteil einstückig ausgebildet sind (Anspruch 4). Um der Unterlage eine hohe Stabilität zu verleihen, kann es vorteilhaft sein, dass sie auf der Rückseite eine in etwa in der Längsmittellinie verlaufende Verstärkungsschiene oder ähnliches aufweist. (Anspruch 5)

Der Patient wird auf der Unterlage fixiert. Um dieses zu unterstützen, ist es vorteilhaft, wenn der Körperteil in seinen seitlichen Bereichen jeweils eine längliche, schlitzförmige Ausnehmung zum Befestigen jeweils eines Gurtteils eines Anschnallgurtes aufweist (Anspruch 6). An dieser Ausnehmung werden Hälften eines Anschnallgurtes befestigt, die auf der Höhe des Thorax des auf dem Rücken liegenden Patienten zusammengeschnallt werden. Im Bereich des Körperteiles kann die flächenartig ausgebildete Unterlage auf einem Operationstisch oder einer ähnlichen Basis aufgelegt werden. Es erscheint aber vorteilhaft, wenn am Kopfteil Mittel zur Verbindung mit einem Operationstisch vorgesehen sind (Anspruch 7). Diese Mittel können vorteilhafterweise als zumindest stückweise umlaufende Auskragung am Kopfteil ausgebildet sein, die in ein entsprechendes, am Operationstisch ausgebildetes oder angeordnetes Gegenstück einklickbar ist (Anspruch 8). Zusätzlich oder alternativ kann die Unterlage aber auch eine unter dem Kopfteil ansetzbare bzw. abnehmbare Stützvorrichtung zum Abstützen des Kopfteiles auf einem Operationstisch aufweisen (Anspruch 9).

Speziell für Schulteroperationen hat es sich als vorteilhaft erwiesen, wenn die Unterlage im Bereich des Thoraxteiles an beiden Seiten ansetzbare bzw. abnehmbare Stützelemente aufweist. Dies erlaubt dem Operateur einerseits einen erleichterten Zugang zur dorsalen Schulter, wenn die Stützelemente entfernt sind und andererseits wird, insbesondere, wenn der Zugang nicht erforderlich ist, eine erweiterte Unterstützung gewährleistet (Anspruch 10).

Um eine optimale Anpassung verschiedener Kopfgrössen zu gewährleisten, ist die aufblasbare Auflage des Kopfteiles nach Anspruch 11 ringförmig ausgebildet. Auch erscheint es vorteilhaft, wenn die aufblasbare Auflage des Halsteiles in etwa trapezförmig ausgebildet ist (Anspruch 12).

Vorteilhaft ist es, dass die aufblasbaren Auflagen des Kopfteiles und des Halsteiles abnehmbar sind, damit sie hygienisch versorgt werden können. Besonders vorteilhaft ist es, wenn sie mit einem Klettverschluss am Kopfteil bzw. am Halsteil anordbar sind. (Anspruch 12).

Vorteilhaft ist es, wenn die abnehmbare, aufblasbare Auflage des Kopfteiles und/oder die abnehmbare, aufblasbare Auflage des Halsteiles einen Klettverschluss aufweist, mit dem sie am Kopfteil bzw. am Halsteil der Unterlage anordbar sind. (Anspruch 13). Auch ist es für den Einsatz zur Vorbereitung zum, beim oder nach dem Operieren vorteilhaft, wenn der Kopfteil, der Halsteil und der Körperteil überwiegend aus einem für Röntgenstrahlen durchlässigen Kunststoff gefertigt sind. (Anspruch 14)

Es sollte in diesem Zusammenhang bemerkt werden, dass die Bezeichnungen Rückenteil, Thoraxteil und Nackenteil die ungefähre Position des menschlichen Körpers angeben, der auf diesen Elementen gelagert wird. Eine genaue anatomische Abgrenzung ist damit nicht gemeint. Vielmehr nimmt auch der obere Bereich des Rückenteils noch menschliche Körperteile auf, in denen sich noch der Thorax befindet. Weiterhin ist es durchaus möglich, dass der Thoraxbereich der Unterlage auch untere Bereiche des menschlichen Halses aufnimmt, insbesondere solche, die der positionierten Unterstützung durch die aufblasbare Auflage des Halsbereiches nicht bedürfen. Es sollte dabei bedacht werden, dass die Dimensionierung des menschlichen Körpers individuell sehr unterschiedlich ist und dass mit einer einzigen Unterlage gemäss der vorliegenden Erfindung Menschen unterschiedlicher Grösse und Breite gelagert werden sollen und können.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können.

### Kurze Beschreibung der Zeichnungen

Ausführungsbeispiele der Erfindung werden nachfolgend anhand der Zeichnungen näher beschrieben, dabei zeigen:
- Figur 1: eine einstückig ausgebildete Unterlage gemäss einem ersten Aus- führungsbeispiel der vorliegenden Erfindung, von der Seite;
- Figur 2: die Unterlage nach Figur 1, von oben;
- Figur 3: die Unterlage nach Figur 1, von unten,
- Figur 4: die Unterlage nach Figur 1, von hinten;
- Figur 5: eine Auflage für den Kopfteil der Unterlage nach Figur 1, von oben, mit den Ausblaselementen;
- Figur 6: die Auflage nach Figur 5, von der Seite;
- Figur 7: eine Auflage für den Halsteil der Unterlage nach Figur 1, von oben, mit den Ausblaselementen;
- Figur 8: die Auflage nach Figur 7, von der Seite;
- Figur 9: eine weitere einstückig ausgebildete Unterlage gemäss einem zweiten Ausführungsbeispiel der vorliegenden Erfindung, von oben;
- Figur 10: ein abnehmbares Stützelement für den Schulterbereich für die Un- terlage nach Figur 9;
- Figur 11: einen Schnitt XI-XI des Verbindungsbereichs zwischen dem Stütz- element und dem Schulterbereich der Unterlage nach Figur 10;
- Figur 12: eine Darstellung der Unterlage, aufgelegt auf den Operationstisch, mit dem Kopfteil eingeklickt und einem Patienten (angedeutet) und

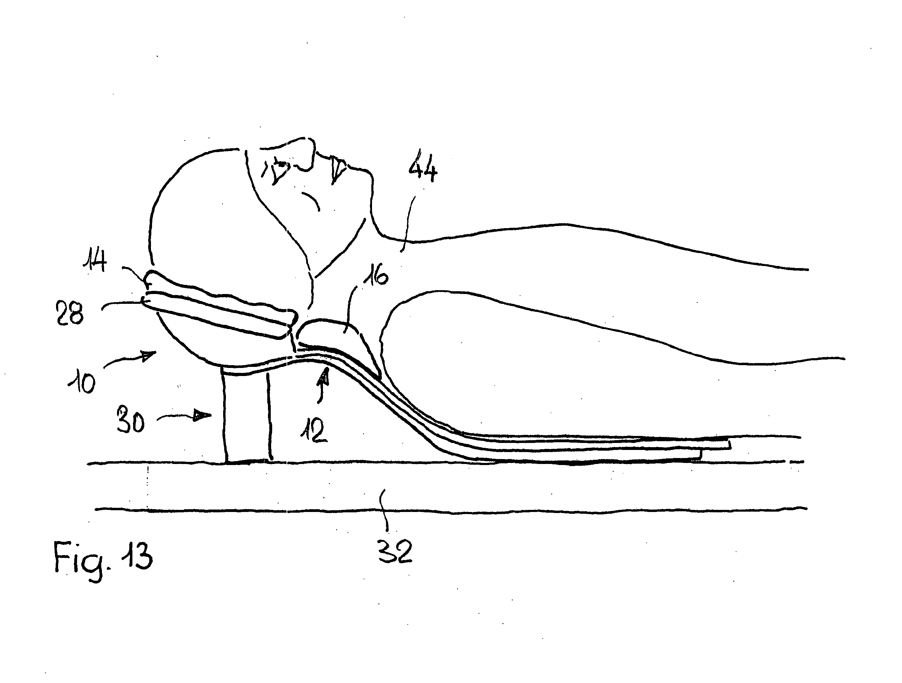

- Figur 13: eine Darstellung der Unterlage, aufgelegt auf den Operationstisch, mit dem Kopfteil aufgelegt auf einem Stützteil.

### Wege zur Ausführung der Erfindung

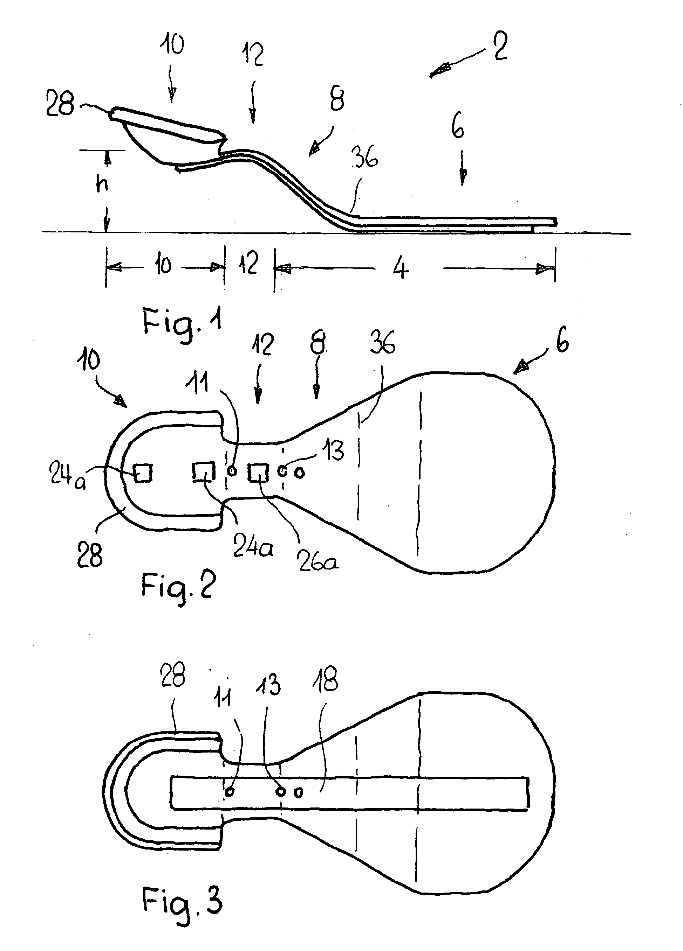

Die in den Figuren 1 bis 4 abgebildete Unterlage 2 zum Lagern eines Patienten bei medizinischen Untersuchungen und chirurgischen Eingriffen, insbesondere bei chirurgischen Operationen im Bereich des Schulterbereichs weist einen im wesentlichen flächenartig ausgebildeten Körperteil 4 auf, der so ausgebildet ist, dass ein Rückenteil 6 in einen dazu verjüngten Thoraxteil 8 übergeht. Die Bezeichnungen Rückenteil und Thoraxteil geben die ungefähre Position des menschlichen Körpers 40 an, wenn dieser auf der Unterlage aufliegt. An den Thoraxteil 8 schliesst sich über einen Übergangsbereich ein Kopfteil 10 an. Dabei ist der Übergangsteil als Halsteil 12 ausgebildet.

Der Thoraxteil 8 weist einen gegenüber dem im Wesentlichen ebenen Rückenteil 6 gerundeten Übergangsbereich 36 auf, durch den der dem Übergangsbereich zum Kopfteil 10 zugewandte Teil des Thoraxteils 8 gegenüber dem dem Rückenteil zugewandten Teil des Thoraxteils 8 nach oben abgewinkelt ist. Dadurch wird erreicht, dass der Kopfteil 10 bei einer Auflage der Unterlage 2 z.B. auf einem Operationstisch 32 um die Höhe h erhöht ist.

Der Kopfteil 10 ist konvex der typischen Kopfform eines erwachsenen Menschen angepasst. Der Rand - ausser in der Nachbarschaft zum Übergangsteil - weist im hier beschriebenen Ausführungsbeispiel eine Auskragung 28 auf, die den Zweck hat, dass der Kopfteil und damit die Unterlage zumindest justiert - vorzugsweise sogar einklickbar oder einrastbar- beispielsweise mit einem bekannten, einstellbaren Kopfhalteteil 36 eines bekannten Operationstisches 32 verbunden werden kann.

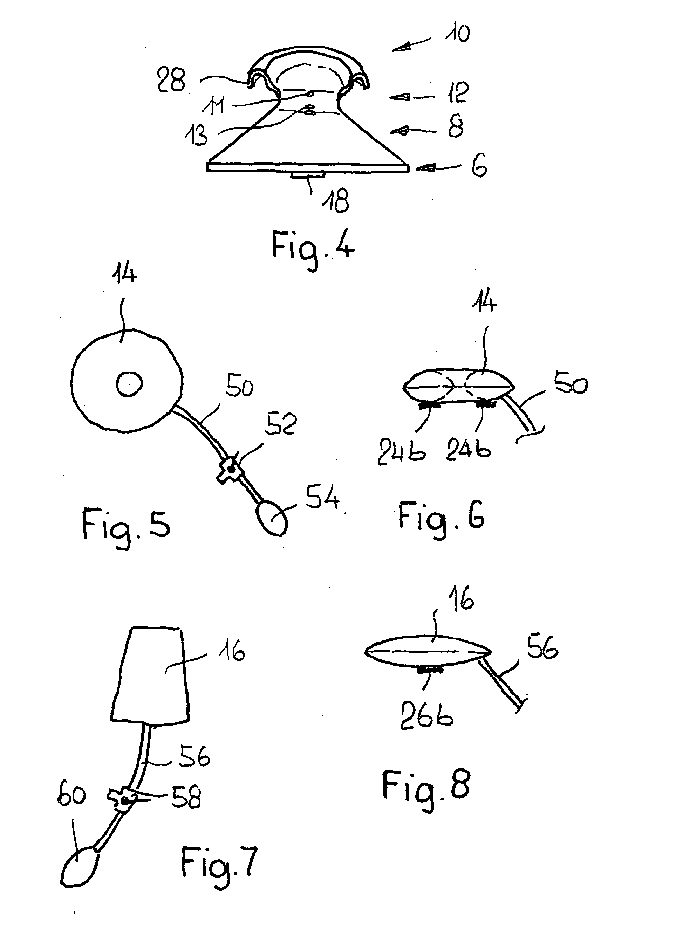

Der Kopfteil 10 ist im vorliegenden Ausführungsbeispiel mit einer ringförmig ausgebildeten Auflageteil 14 belegt, der mittels zweier flächigen Klettverschlüsse 24a und 24b auf dem Kopfteil 10 angeordnet sind. Der Auflageteil 14 ist so ausgebildet, dass der Kopf 42 eines Patienten auf dem Auflageteil ruhen kann und die konvexe Form des Kopfteiles im Wesentlichen dazu dient, die Position des Kopfes 42 bei Bewegungen zu sichern. Wie in den Figuren 5 und 6 dargestellt, ist die Auflage 14 für den Kopfteil 10 mit einem durch eine Öffnung 11 in der Unterlage 2 zu führenden Schlauch 50, einem Dreiwegeventil 52 und einer Handpumpeinrichtung 54 ausgerüstet, damit der Auflageteil 14 für den Kopfteil 10 vor, während und/oder nach einer Operation den Erfordernissen, der Kopfform und der Lage des Patienten und/oder der Behandlungsart, angepasst werden kann.

Der Halsteil 12, der auch gleichzeitig den Übergang zwischen dem Körperteil 4 und dem Kopfteil 10 darstellt, ist als eine Fläche ausgebildet, die dem typischen Hals 44 eines erwachsenen Menschen angepasst ist. Da dies aber zumindest dann schwierig ist, wenn die Lage des Patienten und damit die Lage und Ausrichtung seines Halses 44 nicht eindeutig vorbestimmt werden kann. Um dieses Problem zu lösen, ist im vorliegenden Ausfünrungsbeispiel auch der Halsteil 12 mit einem vorzugsweise trapezförmig ausgebildeten Auflageteil 16 belegt, der mittels eines flächigen Klettverschlusses 26a und 26b auf dem Halsteil 12 angeordnet sind. Damit kann eine ungestützte Positionierung des Halses 44 in jeder Lage und bei jeder Bewegung des Patienten vermieden werden. Insbesondere ist es so möglich, den Kopf und den Hals des Patienten in eine für die Behandlung zum Beispiel zum Intubieren geeignete und stabile Lage zu bringen.

Wie in den Figuren 7 und 8 dargestellt, ist auch die Auflage 16 für den Halsteil 12 mit einem durch eine Öffnung 13 in der Unterlage 2 zu führenden Schlauch 56, einem Dreiwegeventil 58 und einer Handpumpeinrichtung 60 ausgerüstet, damit der Auflageteil 16 für den Halsteil 12 vor, während und nach einer Operation den Erfordernissen, insbesondere der gewünschten Lage und Behandlungsform angepasst werden kann.

Für den Fall, dass der Kopfteil 10 nicht mit dem entsprechenden einstellbaren Kopfhalteteil 36 des Operationstisches 32 verbunden ist, kann eine Stützvorrichtung 30 vorgesehen sein, mit der der Kopfteil 10 so abgestützt wird, dass die Unterlage 2 kippsicher auf einer Fläche aufgelegt werden kann.

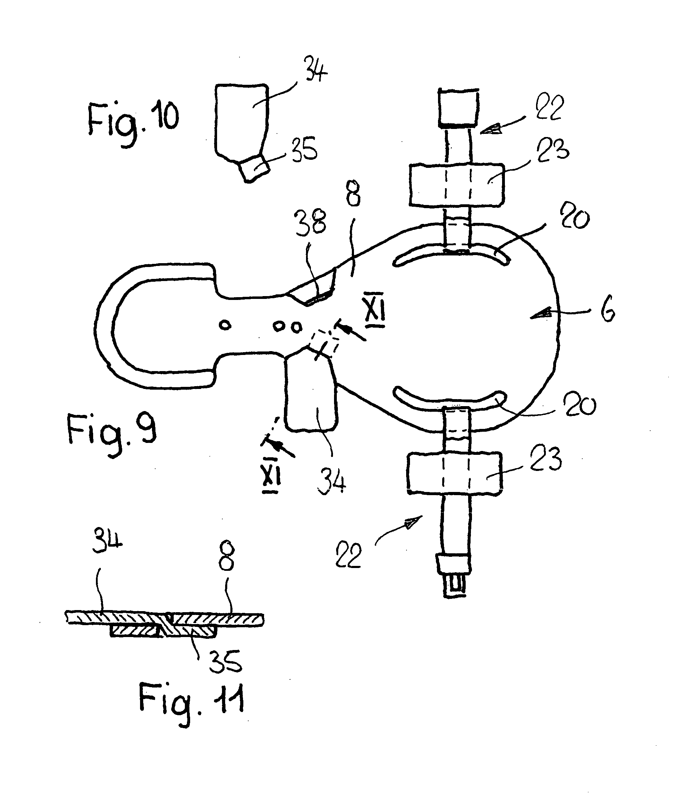

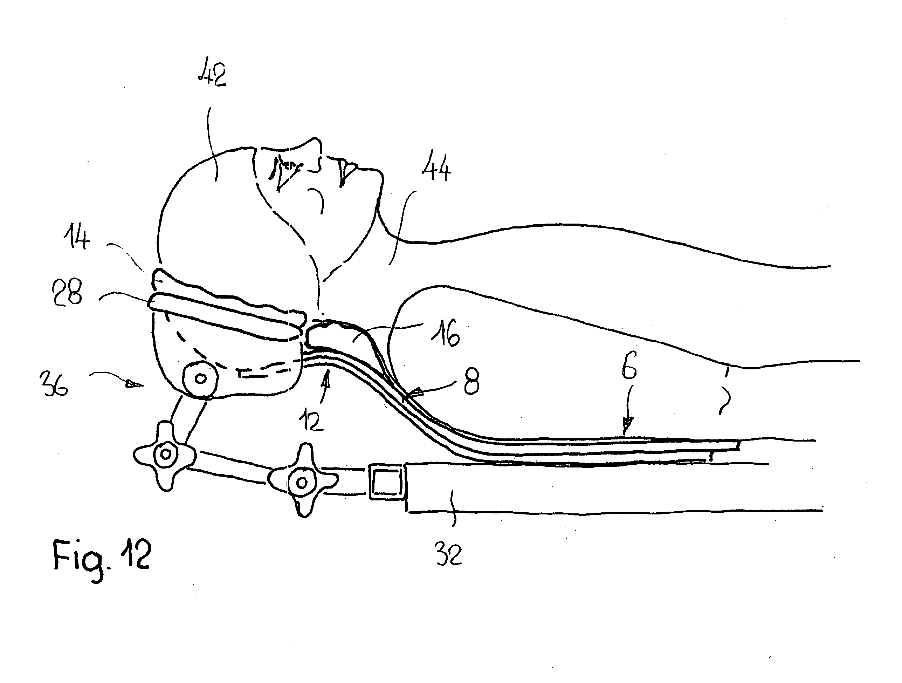

In den Figur 9 bis 12 werden Abwandlungen der vorstehend beschriebenen Ausführungsform gezeigt. Die in Figur 9 gezeigte Unterlage weist in einem bestimmten Abstand zu den seitlichen Rändern des Körperteils 6 jeweils schlitzförmige Ausnehmungen 20 auf, die zum Befestigen jeweils einer Gurthälfte eines Anschnallgurtes 22 dienen, mit denen der auf der Unterlage 2 positionierte Patient auf der Unterlage 2 angeschnallt werden kann. An dem Anschnallgurt 22 sind vorzugsweise eine oder mehrere Stützplatten 23 angeordnet, um die Haltekraft des Anschnallgurtes flächig auf dem Körper des Patienten zu verteilen. Grundsätzlich ist es damit möglich, dass der Patient mitsamt der Unterlage auf den Operationstisch 32 gelagert, weiter in eine "beach chair Position" bis in eine Sitzposition gebracht werden kann. Ferner kann der Patient mittels der Unterlage vom Operationstisch angenommen und umgelagert, allenfalls sogar unabhängig davon transportiert werden.

Der Thoraxteil 8 weist seitlich jeweils Aufnahmeschlitze 38 Einrasten von Zungen 35 von Stützelementen 34 auf, wie dies insbesondere in den Figuren 9 bis 11 gezeigt ist. Die abnehmbaren Stützelemente 34 dienen dazu, im Bedarfsfalle, z.B. beim Bohren im Schulterbereich des Patienten, eine ausreichende Gegenfläche auszubilden. Sie sind abnehmbar, um den dorsalen Zugang zum Gelenk, wenn der Bedarf nicht besteht, die Möglichkeit, den Patienten zu bewegen, zu gewährleisten bzw. zu erhöhen. Die Stützelemente werden eingeschoben und - wie in Figur 11 gezeigt - in den Thoraxteil eingerastet.

Gegebenenfalls kann der Kopfteil 10 nicht einstückig mit dem Körperteil 4 und dem Halsteil 12 ausgebildet sein, sondern an letzteren anmontiert, z.B. abnehmbar angeschraubt sein. Dies erleichtert z.B. den Austausch des Kopfteiles 10, wenn Patienten anderer Dimension operiert werden sollen, z.B. Kinder oder Jugendliche.

Die Unterlage wird bei Operationen, insbesondere bei Schulteroperationen in der so genannten "beach chair" Position, eingesetzt. Dabei ist es vorteilhaft, wenn während der Operation oder aber vor und nach der Operation, wenn der Patient noch auf der Unterlage angeschnallt ist, Untersuchungen mit Röntgenstrahlen durchgeführt werden können. Aus diesem Grund ist die Unterlage, insbesondere der Kopfteil 10, der Halsteil 12 und der Körperteil 4 aus einem für Röntgenstrahlen durchlässigen Kunststoff gefertigt.

Im vorliegenden Ausführungsbeispiel ist die Unterlage 2 aus Kunststoff. Um der Unterlage eine erhöhte Festigkeit, insbesondere gegen Verbiegungen und Torsionen zu verleihen, ist der Körperteil 4 und der Halsteil 12 der Unterlage 2 auf der Rückseite mit einer Verstärkungsschiene 18 ausgestattet.

Im Folgenden wird eine typische Verwendung der erfindungsgemässen Unterlage beschrieben. Wenn der auf der Unterlage positionierte und angeschnallte Patient operiert wird, so wird ihm zunächst ein Schlafmittel verabreicht. Er schläft also und wird beatmet. Da die Hals- und Brustwirbelsäule nicht unterstützt sind, würde sie ohne Hilfsmittel nach hinten fallen. Mit der Unterlage gemäss der vorstehenden Beschreibung wird eine Stabilisierung des Halses ermöglicht, dadurch fällt er nicht nach hinten und mit dem Aufblasen der trapezförmigen Auflage 16 kann der Hals nach Belieben nach vorne gedrückt werden, so dass sich der Kopf von selbst in den Nacken legt.

Die Unterlage ist sehr vielfältig einsetzbar, wobei sie in jedem Falle eine einwandfrei Unterstützung des Patienten vom Kopf über den Hals und den Schulter und Brustteil bis zum Rücken gewährleistet. Dies ist insbesondere im Falle der Narkose besonders wichtig, da dann die Muskulatur des Patienten entspannt ist und an nicht unterstützen Stellen der Körper durchhängen und Schaden nehmen könnte.

Beim Umlagern eines Patienten auf den Operationstisch, also vor seiner Narkotisierung, wird festgelegt, in welcher Stellung und mit welchem Druckniveau der aufblasbaren Auflagen 14 und 16 er bequem liegt und entsprechend werden die Auflagen 14 und 16 aufgeblasen. Nach einer Lageveränderung, z.B. einer Intubation, würde man zudem sehen, wenn die entsprechende Unterstützung durch den Halsteil 12 und seine Auflage 16 fehlt.

Zum Intubieren, das heisst zum Einführen eines Schlauches in die Luftröhre eines Patienten kann es erforderlich sein, dass auch der Kopf nach vorne gedrückt wird, um eine so genannte Schnüffel- oder Jacksonstellung einzunehmen. Hierzu wird die ringförmige Auflage 14 weiter aufgeblasen. Nach der Intubation wird die Luft aus dieser Auflage 14 wieder auf ein zuvor vom Patienten bestimmtes Niveau abgelassen.

Wenn der Patient - nach der Intubation - aufgesetzt werden soll, besteht bei herkömmlicher Einrichtungen die Gefahr, dass sich der Kopfteil verschiebt. Dies wird durch die den Einrastmechanismus der Auskragung 28 in den Kopfhalteteil des Operationstisches 36 verhindert. Der Kopfteil 10 kann nicht ausscheren. Die Sicherheit wird noch durch die Verstärkungsschiene erhöht, durch die die Unterlage 2 - im Ausführungsbeispiel einschliesslich des Halsteiles 12 - versteift ist.

### Bezugszeichenliste

- 2: Unterlage
- 4: Körperteil
- 6: Rückenteil
- 8: Thoraxteil
- 10: Kopfteil
- 11: Öffnung
- 12: Halsteil
- 13: Öffnung
- 14: Auflage Kopfteil
- 16: Auflage Halsteil
- 18: Verstärkungsschiene
- 20: Schlitzförmige Ausnehmungen zur Aufnahme eines Anschnallgurtes
- 22: Anschnallgurt
- 23: Stützplatte
- 24a,b: Klettverschluss Kopfteil
- 26a,b: Klettverschluss Halsteil
- 28: Auskragung Kopfteil
- 30: Stützvorrichtung Kopfteil
- 32: Operationstisch
- 34: abnehmbare Stützelemente
- 35: Lasche
- 36: Kopfhalteteil des Operationstisches
- 38: Aufnahmeeinschnitte für die Stützelemente
- 40: Körper
- 42: Kopf
- 44: Hals
- 50: Schlauch
- 52: Dreiwegeventil
- 54: Handpumpeinrichtung
- 56: Schlauch
- 58: Dreiwegeventil
- 60: Handpumpeinrichtung

## Patentansprüche

1. Unterlage (2) zum Lagern eines Patienten, mit einem im wesentlichen flächenartig ausgebildeten Körperteil (4) und einem schalenartigen Kopfteil (10), wobei der Körperteil (4) einen Rückenteil (6) und einen dazu verjüngten Thoraxteil (8) aufweist, an dem der Kopfteil (10) mittels eines Übergangsbereiches angeordnet ist, wobei der Thoraxteil (8) und der Übergangsbereich ansteigend derart ausgebildet sind, dass der Kopfteil (10) höher ist als der Rückenteil (6) und wobei der Kopfteil (10) den menschlichen Umrissformen angepasst sind und eine abnehmbare, aufblasbare Auflage (14) zur Anpassung der Lage des Patienten an die Lage- bzw. Behandlungserfordernisse aufweist, wobei der Übergangsbereich als Halsteil (12) ausgebildet ist und ebenfalls den menschlichen Umrissformen angepasst sind und eine abnehmbare aufblasbare Auflage (16) zur Anpassung der Lage des Patienten an die Lage- bzw. Behandlungserfordernisse aufweist.

2. Unterlage nach Anspruch 1, **dadurch gekennzeichnet, dass** der Halsteil (12) in seiner Länge mindestens der Länge des menschlichen Halses (44) und in seiner Breite mindestens der halben Breite des menschlichen Halses (44) entspricht.

3. Unterlage nach Anspruch 1 oder 2, **dadurch gekennzeichnet, dass** der Körperteil (4) den menschlichen Umrissen angepasst ist.

4. Unterlage nach einem der Ansprüche 1 bis 3, **dadurch gekennzeichnet, dass** zumindest der Körperteil (4) und der Halsteil (12) einstückig ausgebildet sind.

5. Unterlage nach einem der Ansprüche 1 bis 4, **dadurch gekennzeichnet, dass** die Unterlage (2) auf der Rückseite eine in etwa in der Längsmittellinie verlaufende Verstärkungsschiene (18) aufweist.

6. Unterlage nach einem der Ansprüche 1 bis 5, **dadurch gekennzeichnet, dass** der Körperteil (4) in seinen seitlichen Bereichen jeweils eine längliche schlitzförmige Ausnehmung (20) zum Befestigen jeweils eines Gurtteils eines Anschnallgurtes (22) aufweist.

7. Unterlage nach einem der Ansprüche 1 bis 6, **dadurch gekennzeichnet, dass** am Kopfteil (10) Mittel zur Verbindung mit einem Operationstisch aufweist.

8. Unterlage nach Anspruch 7, **dadurch gekennzeichnet, dass** die Mittel zur Verbindung am Kopfteil (10) als zumindest stückweise umlaufende Auskragung (28) ausgebildet sind, die in ein entsprechendes, am Operationstisch ausgebildetes oder angeordnetes Gegenstück einrastbar ist.

9. Unterlage nach einem der Ansprüche 1 bis 8, **dadurch gekennzeichnet, dass** sie eine unter dem Kopfteil (10) ansetzbare bzw. abnehmbare Stützvorrichtung (30) zum Abstützen des Kopfteiles auf einem Operationstisch aufweist.

10. Unterlage nach einem der Ansprüche 1 bis 9, **dadurch gekennzeichnet, dass** sie im Bereich des Thoraxteiles (8) an beiden Seiten ansetzbare bzw. abnehmbare Stützelemente (34), insbesondere für den Schulterbereich des Patienten aufweist.

11. Unterlage nach einem der Ansprüche 1 bis 10, **dadurch gekennzeichnet, dass** die aufblasbare Auflage (14) des Kopfteiles (10) ringförmig ausgebildet ist.

12. Unterlage nach einem der Ansprüche 1 bis 11, **dadurch gekennzeichnet, dass** die aufblasbare Auflage (16) des Halsteiles (12) in etwa trapezförmig ausgebildet ist.

13. Unterlage nach einem der Ansprüche 1 bis 12, **dadurch gekennzeichnet, dass** die abnehmbare, aufblasbare Auflage (14) des Kopfteiles (10) und/oder die abnehmbare, aufblasbare Auflage (16) des Halsteiles (12) einen Klettverschluss aufweist, mit dem sie am Kopfteil (10) bzw. am Halsteil (12) anordbar ist.

14. Unterlage nach einem der Ansprüche 1 bis 13, **dadurch gekennzeichnet, dass** der Kopfteil (10), der Halsteil (12) und der Körperteil (4) überwiegend aus einem für Röntgenstrahlen durchlässigen Kunststoff gefertigt ist.

## Claims

1. A support (2) for supporting a patient, with a substantially flat body part (4) and a bowl-shaped head part (10), the body part (4) having a back part (6) and a thorax part (8) which is tapered in relation thereto and on which the head part (10) is arranged by means of a transitional region, the thorax part (8) and the transitional region rising in such a way that the head part (10) is higher than the back part (6) and the head part (10) being adapted to the human profile and having a detachable, inflatable rest (14) for adapting the position of the patient to the positional or treatment requirements, wherein the transitional region is embodied as a neck part (12) and is also adapted to the human profile and has a detachable, inflatable rest (16) for adapting the position of the patient to the positional or treatment requirements.

2. The support as claimed in claim 1, **characterized in that** the neck part (12) corresponds in its length at least to the length of the human neck (44) and in its width at least to half the width of the human neck (44).

3. The support as claimed in claim 1 or 2, **characterized in that** the body part (4) is adapted to the human profile.

4. The support as claimed in one of claims 1 to 3, **characterized in that** at least the body part (4) and the neck part (12) are embodied in one piece.

5. The support as claimed in one of claims 1 to 4, **characterized in that** the support (2) has on the back a reinforcement rail (18) extending substantially in the longitudinal center line.

6. The support as claimed in one of claims 1 to 5, **characterized in that** the body part (4) has in each of its lateral regions an elongate, slotted recess (20) for fastening a respective belt part of a safety belt (22).

7. The support as claimed in one of claims 1 to 6, **characterized in that** the head part (10) has means for connecting to an operating table.

8. The support as claimed in claim 7, **characterized in that** the means for connecting to the head part (10) are embodied as a projection (28) which encircles at least in certain pieces and can be locked into a corresponding counterpiece embodied or arranged on the operating table.

9. The support as claimed in one of claims 1 to 8, **characterized in that** it has a support device (30), which can be attached or detached below the head part (10), for supporting the head part on an operating table.

10. The support as claimed in one of claims 1 to 9, **characterized in that** it has support elements (34) which can be attached or detached in the region of the thorax part (8) at both sides, in particular for the shoulder region of the patient.

11. The support as claimed in one of claims 1 to 10, **characterized in that** the inflatable rest (14) of the head part (10) is embodied in an annular manner.

12. The support as claimed in one of claims 1 to 11, **characterized in that** the inflatable rest (16) of the neck part (12) is embodied in a substantially trapezoidal manner.

13. The support as claimed in one of claims 1 to 12, **characterized in that** the detachable, inflatable rest (14) of the head part (10) and/or the detachable, inflatable rest (16) of the neck part (12) has a hook-and-loop fastener which can be used to arrange the rest on the head part (10) or on the neck part (12).

14. The support as claimed in one of claims 1 to 13, **characterized in that** the head part (10), the neck part (12) and the body part (4) are made predominantly of a plastics material which is transparent to x-rays.

## Revendications

1. Support (2) pour positionner un patient comprenant une partie de corps (4) essentiellement plate et une partie de tête (10) essentiellement en forme de coquille,
- la partie de corps (4) comporte une partie de dos (6) et une partie de thorax (8) allant en se rétrécissant et à laquelle est reliée la partie de tête (10) par une zone transitoire,
- la partie de thorax (8) et la zone transitoire étant inclinées de façon que la partie de tête (10) soit plus haute que la partie de dos (6), et
- la partie de tête (10) est adaptée aux formes de contour du corps humain et comporte un appui (14) gonflable, amovible, pour adapter la position du patient à la position ou aux nécessités de l'intervention,
- la zone transitoire étant réalisée comme partie de cou (12) en étant également adaptée aux formes de contour du corps humain et comporte un appui gonflable amovible (16) pour adapter la position du patient aux nécessités de positionnement et de l'intervention.

2. Support selon la revendication 1,
**caractérisé en ce que**
la partie de cou (12) a sa longueur adaptée au moins à la longueur du cou du corps humain (44) et sa largeur correspond au moins à la demi-largeur du cou du corps humain (44).

3. Support selon la revendication 1 ou 2,
**caractérisé en ce que**
la partie de corps (4) est adaptée au contour des parties du corps humain.

4. Support selon l'une des revendications 1 à 3,
**caractérisé en ce qu'**
au moins la partie de corps (4) et la partie de cou (12) sont réalisées en une seule pièce.

5. Support selon l'une des revendications 1 à 4,
**caractérisé en ce que**
sur le côté du dos, le support (2) comporte un rail de renforcement (18) s'étendant sensiblement suivant l'axe longitudinal.

6. Support selon l'une des revendications 1 à 5,
**caractérisé en ce que**
dans ses zones latérales, la partie de corps (4) comporte respectivement une découpe longitudinale (20) en forme de fente pour fixer une partie d'une ceinture d'attache (22).

7. Support selon l'une des revendications 1 à 6,
**caractérisé en ce que**
la partie de tête (10) comporte des moyens pour être reliée à une table d'opération.

8. Support selon la revendication 7,
**caractérisé en ce que**
les moyens de fixation de la partie de tête (10) sont réalisés au moins partiellement sous la forme de rebords (28) périphériques, qui sont enclipés dans une pièce complémentaire réalisée sur la table d'opération ou installée sur celle-ci.

9. Support selon l'une des revendications 1 à 8,
**caractérisé en ce qu'**
il comporte un dispositif d'appui (30), amovible ou qui s'installe sous la partie de tête (10) pour soutenir la partie de tête sur la table d'opération.

10. Support selon l'une des revendications 1 à 9,
**caractérisé en ce que**
dans la zone de la partie de thorax (8), il comporte des éléments de support (34) amovibles ou qui s'installent sur les deux côtés, notamment au niveau de la région des épaules du patient.

11. Support selon l'une des revendications 1 à 10,
**caractérisé en ce que**
le support gonflable (14) de la partie de tête (10), a une forme annulaire.

12. Support selon l'une des revendications 1 à 11,
**caractérisé en ce que**
le support gonflable (16) de la partie de cou (12) a une forme sensiblement trapézoïdale.

13. Support selon l'une des revendications 1 à 12,
**caractérisé en ce que**
le support gonflable amovible (14) de la partie de tête (10) et/ou le support gonflable amovible (16) de la partie de cou (12), comportent une liaison à griffes par laquelle le support s'installe sur la partie de tête (10) ou la partie de cou (12).

14. Support selon l'une des revendications 1 à 13,
**caractérisé en ce que**
la partie de tête (10), la partie de cou (12) et la partie de corps (4) sont réalisées principalement en une matière plastique transparente aux rayons X.
